# EUROPÄISCHE PATENTSCHRIFT

(11) **EP 2 858 606 B1**
(45) Veröffentlichungstag und Bekanntmachung des Hinweises auf die Patenterteilung: **02.06.2021**
(21) Anmeldenummer: 13728962.5
(22) Anmeldetag: 06.06.2013
(51) Int. Cl.: A61F 5/01

(54) **ORTHESE**
ORTHOSIS
ORTHÈSE

(30) Priorität: 12.06.2012 DE 102012011467
(43) Veröffentlichungstag der Anmeldung: 15.04.2015
(73) Patentinhaber: Ottobock SE & Co. KGaA, 37115 Duderstadt (DE)
(72) Erfinder: LJUBIMIR, Boris, 70825 Korntal-Münchingen (DE); SCHMITT, Alexander, 34123 Kassel (DE)
(74) Vertreter: Stornebel, Kai
(86) Internationale Anmeldenummer: PCT/EP2013/001665
(87) Internationale Veröffentlichungsnummer: WO 2013/185895

(56) Entgegenhaltungen:
- WO-A1-02/083040
- WO-A1-2009/092348
- US-A- 2 712 310
- US-A- 5 584 072
- US-A1- 2011 306 911

## Beschreibung

Die Erfindung betrifft eine Orthese mit einem Abstützbereich, der Befestigungsmittel aufweist, um die Orthese an einer Gliedmaße anzulegen, wobei der Abstützbereich einander entgegengesetzt orientierte Aufnahmebereiche aufweist, die medial und lateral vorgesehen sind..

Orthesen dienen zur Stabilisierung von Gliedmaßen und können gelenkübergreifend angeordnet sein. Orthesen dienen unter anderem zur Sicherstellung oder Unterstützung eines gewünschten Bewegungsablaufes, zur Unterstützung des Stützapparates, zur Begrenzung des Bewegungsumfanges von Gelenken oder zur Beaufschlagung einer Gliedmaße mit einer bestimmten Kraft. Zur Festlegung einer Orthese an einer Gliedmaße werden Befestigungsmittel eingesetzt, die in der Regel als Riemen oder Gurt ausgebildet sind. Diese Riemen oder Gurte sind an Rahmenteilen befestigt, beispielsweise angeklebt oder vernietet.

Die WO 2009/092348 A1 betrifft eine Armabduktionsorthese mit einem zur Abstützung an dem Thorax eines Orthesennutzers vorgesehenen Körperrahmen, an dem Befestigungsmittel zur Fixierung des Körperrahmens an dem Orthesennutzer angeordnet sind. Eine Auflageeinrichtung zur Auflage eines Armes des Orthesennutzers ist gelenkig an dem Körperrahmen befestigt. Der Rahmen wird über Befestigungsmittel an der Auflageeinrichtung festgelegt. Die Auflageeinrichtung weist einen Abstützurahmen mit zwei zueinander beabstandeten Rahmenprofilen auf, zwischen denen ein Auflagematerial angeordnet ist, wobei sich der Abstützrahmen an dem Körperrahmen über zumindest ein Stützelement abstützt.

Die US-A-5,584,072 betrifft einen Hüftschutz mit einem Hüftgurt und einem Oberschenkelgurt, die über eine Polsteraufnahme miteinander verbunden sind. In der Polsteraufnahme können weiche Polsterkissen eingearbeitet oder eingesteckt sein.

Die US 2011/0306911 A1 betrifft eine Knieorthese mit medial und lateral angeordneten Stützschienen, die jeweils zwei gelenkig miteinander verbundene Schenkel aufweisen. Die Stützschienen sind auf der Außenseite eines elastischen Grundkörpers in Taschen festgelegt, deren Öffnung einander gegenüberliegen.

Die WO 02/083040 A1, die den Oberbegriff des Anspruchs 1 offenbart, betrifft eine Knöchel-Fuß-Orthese mit einem Fußteil, in das sich nach oben erstreckenden Strebe, die mit der Fußplatte verbunden ist sowie Befestigungsmittel, zum Befestigen der Orthese an einem Bein. Die Strebe gabelt sich in zwei Äste, die auf beiden Seiten des Schienbeins angeordnet sind. Über einen Riemen, der über einen Klettverschluss an den beiden oberen Enden der Äste festgelegt wird, wird die Orthese an dem Bein gehalten. Der Riemen wird auf sich selbst fixiert.

Die US 2,712,310 A betrifft eine Orthese bei einem Fall-Fuß-Syndrom mit einer Fußschale, die in einem Schuh getragen wird, von der aus sich zwei seitliche Federstäbe medial und lateral an einem Bein entlang erstrecken. Die oberen Enden der Federstäbe sind in nach unten geöffneten Taschen eingeführt, die auf einem einen unterschenkelumschließenden Gurt angenäht sind.

Aufgabe der vorliegenden Erfindung ist es, eine Orthese bereitzustellen, die einfach herzustellen und zu reinigen ist und eine sichere Festlegung der Orthese an der Gliedmaße gewährleistet.

Erfindungsgemäß wird diese Aufgabe durch eine Orthese mit den Merkmalen des Hauptanspruches gelöst. Vorteilhafte Ausgestaltungen und Weiterbildungen der Erfindung sind in den Unteransprüchen, der Beschreibung und den Figuren offenbart. Die erfindungsgemäße Orthese mit einem Abstützbereich, der Befestigungsmittel aufweist, um die Orthese an einer Gliedmaße anzulegen, sieht vor, dass der Abstützbereich einander entgegengesetzt orientierte, medial und lateral vorgesehene Aufnahmebereiche, z.B. in Gestalt von Vorsprüngen, aufweist und dass an dem Befestigungsmittel Taschen zur Aufnahme der Aufnahmebereiche angeordnet sind. Die Taschen sind über ein reversibel festlegbares Verbindungselement miteinander verbunden, bevorzugt ist dieses Verbindungselement über den Abstützbereich geführt, so dass ein Herunterrutschen der Taschen und damit des Befestigungsmittels von den Aufnahmebereichen verhindert wird. Durch die einander entgegengesetzt orientierten Aufnahmebereiche oder Vorsprünge werden Formschlusselemente an dem formstabilen Abstützbereich der Orthese ausgebildet, die in Taschen eingeführt werden können, die an dem Befestigungsmittel angeordnet sind, wobei das Verbindungselement als ein den Abstützbereich und die Gliedmaße umschlingender Gurt ausgebildet ist. Die Taschen ermöglichen einerseits eine sichere Festlegung des Befestigungsmittels aufgrund der entgegengesetzten Orientierung der Vorsprünge und andererseits eine einfache Entfernung des Befestigungsmittels von dem Abstützbereich, indem die Aufnahmebereiche aus den Taschen entfernt und das Befestigungsmittel abgenommen werden kann. Dadurch ist es möglich, die Befestigungsmittel beispielsweise zu waschen oder alternative Befestigungsmittel zu verwenden, die beispielsweise an die Form der Gliedmaße besser angepasst sind.

Das Befestigungsmittel ist vorteilhafterweise Teil einer Polsterung oder weist eine Polsterung auf, die auf der dem Polster zugewandten Seite der Orthese angeordnet ist. Dadurch wird einerseits die Festlegung der Orthese an der Gliedmaße gesichert und andererseits die Festlegung der Polsterung an dem Abstützbereich. Die reversible Anordnung der Polsterung an dem Abstützbereich hat den Vorteil, dass auch die Polsterung ausgewechselt und an die Wünsche oder Gegebenheiten des jeweiligen Patienten angepasst werden kann.

Die Taschen sind vorteilhafterweise medial und lateral angeordnet, so dass ein erleichtertes Anlegen der Orthese möglich ist.

Eine Weiterbildung der Erfindung sieht vor, dass die lateral angeordneten Taschen und/oder Aufnahmebereiche länger als die medial angeordneten Taschen und/oder Aufnahmebereiche sind. Dadurch wird gewährleistet, dass bei einer Gutführung von medial nach lateral die in Umfangsrichtung wirkenden Kräfte dazu führen, dass die lateral ausgebildeten Aufnahmebereiche nicht aus den Taschen heraus gleiten können. Die Anordnung mit lateral tieferen Taschen und längeren Aufnahmebereichen bewirkt, dass selbst bei einer Verdrehung in Umfangsrichtung um die Länge der medialen Aufnahmebereiche oder medialen Taschen und die lateralen Aufnahmebereiche weiterhin in den lateralen Taschen verbleiben. Dadurch wird ein Ausziehen der Aufnahmebereiche aus den Taschen vermieden.

Der Abstützbereich ist vorteilhafterweise schalenartig oder rinnenartig ausgebildet, so dass sich eine im Wesentlichen flächige Anlage um die Gliedmaße herum ausgebildet. Durch die schalenartige oder rinnenartige Ausgestaltung ist es möglich, die Orthese einfach auf die Gliedmaße aufzulegen und anschließend über die Befestigungsmittel an der Gliedmaße festzulegen. Die schalenartige oder rinnenartige Ausgestaltung kann mit Durchbrüchen versehen sein, um eine Belüftung zu ermöglichen und gegebenenfalls Gewicht zu sparen.

Eine Weiterbildung der Erfindung sieht vor, dass das Verbindungselement elastisch ausgebildet ist und dadurch eine Vorspannung der Taschen in Richtung aufeinander zu ermöglicht wird. Die Vorspannung wird dann erreicht, wenn vor der Festlegung des Verbindungselementes auf den Taschen das Verbindungselement gedehnt wird. Die Taschenwerden dann auf die Aufnahmebereiche gezogen und dort unter einer Vorspannung gehalten.

Das Verbindungselement kann Teil des Befestigungsmittels sein, beispielsweise kann durch das Verbindungselement eine Tasche ausgebildet sein, beispielsweise eine medial angeordnete Tasche, so dass das Verbindungselement außen über das Abstützelement geführt werden kann. Die Aufnahmebereiche sind dabei zwischen dem Verbindungselement und dem Befestigungsmittel angeordnet und in die durch das Verbindungselement und das Befestigungsmittel ausgebildete Tasche eingeschoben.

Auf der Außenseite zumindest einer Tasche kann eine Fixiereinrichtung für das Verbindungselement angeordnet sein, beispielsweise in Gestalt von Flausch- und Hakenbereichen. Dadurch ist es möglich, dass das Verbindungselement als ein den Abstützbereich und die Gliedmaße umschlingender Gurt ausgebildet sein kann, der gleichzeitig eine Fixierung der Taschen an dem Abstützelement bewirkt. Bei einer elastischen Ausgestaltung des Gurtes kann die Vorspannung der Taschen aufeinander zu über den Gurt gewährleistet sein. Es ist auch vorgesehen, dass das Verbindungselement als ein den Abstützbereich und die Gliedmaße umschlingender Gurt ausgebildet sein kann, ohne dass es über eine Fixiereinrichtung auf der Außenseite einer Tasche festgelegt ist.

Im angelegten Zustand ist vorgesehen, dass das Verbindungselement einen Freiraum zwischen den Aufnahmebereichen von lateral nach medial überbrückt. Die Gurtführung verläuft somit von der medialen Tasche über die laterale Tasche zurück zur medialen Tasche oder dem medialen Vorsprung, wodurch eine vollständige Umschlingung der Gliedmaße erreicht wird. Der Freiraum zwischen den Aufnahmebereichen ist bei einer rinnenartigen oder schalenartigen Ausgestaltung derjenige Raum, in den die Gliedmaße eingeführt wird.

Der Abstützbereich kann in seiner Längserstreckung, die in der Regel der Längserstreckung der Gliedmaße entspricht, elastisch um eine Achse parallel zu der Längserstreckung ausgebildet sein, so dass sich eine Anpassbarkeit in Medialrichtung und Lateralrichtung durch den Abstützbereich realisieren lässt. Darüber hinausgehende Verformungen werden vorteilhafterweise von dem Abstützbereich nicht zugelassen, so dass eine sichere Abstützung des Abstützbereiches und dadurch die Festlegung der Orthese an den Gliedmaßen möglich sind.

Eine Weiterbildung der Erfindung sieht vor, dass die Orthese weiterhin einen Auflagebereich aufweist, der mit dem Abstützbereich verbunden ist und sich an der Gliedmaße abstützt. Der Auflagebereich ist vorteilhafterweise gelenkübergreifend angeordnet, so dass beispielsweise bei einer Fußorthese der Auflagebereich derjenige Bereich ist, auf den der Fuß aufgestellt wird, während der Abstützbereich derjenige Bereich der Orthese ist, der sich an dem Unterschenkel abstützt.

Der Auflagebereich und der Abstützbereich können gelenkig miteinander verbunden sein, alternativ ist vorgesehen, dass eine mehr oder weniger starre Verbindung zwischen beiden Bereichen vorliegt, wobei eine Relativverlagerung des Auflagebereiches zu dem Abstützbereich im Rahmen der Materialelastizität möglich ist.

Die Aufnahmebereiche können als Vorsprünge ausgebildet sein. Die Aufnahmebereiche ermöglichen es, dass die Taschen an der Orthese festgelegt werden, indem diese Bereiche in die Taschen eingeführt werden. Es kann medial und lateral jeweils ein Aufnahmebereich oder Vorsprung oder auch mehrere Aufnahmebereiche oder Vorsprünge vorgesehen sein, so dass entweder eine Tasche oder mehrere Taschen medial und lateral vorhanden sind. Die Aufnahmebereiche erstrecken sich von dem Abstützbereich medial und lateral nach hinten, so dass eine Aufnahme in den Taschen möglich ist. Wenn der Abstützbereich rinnenartig ausgebildet ist und geradwandige Ränder aufweist, sind die Aufnahmebereiche als Vorsprünge ausgebildet, die von den Rändern abstehen und hervorragen. Es ist auch möglich und vorgesehen, dass der jeweilige Aufnahmebereich von einer Ausformung ausgebildet wird, die sich an den Abstützbereich anschließt.

Nachfolgend werden Ausführungsbeispiele der Erfindung anhand der beigefügten Figuren näher erläutert. Es zeigen:
- Figur 1: - eine Orthese mit geschlossenen Verbindungselementen; sowie
- Figur 2: - eine Orthese gemäß Figur 1 mit offenen Verbindungselementen;
- Figur 3 -: eine Variante der Erfindung;
- Figur 4 -: eine Orthese mit dorsalem Abstützbereich; sowie
- Figur 5 -: ein Befestigungsmittel in Einzeldarstellung.

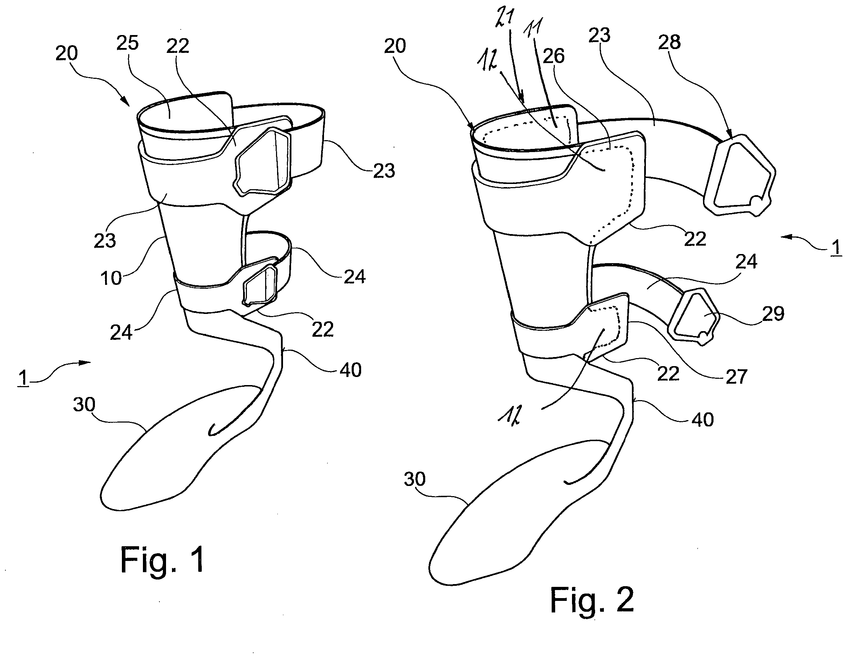

In der Figur 1 ist eine Orthese in Gestalt einer sogenannten Ankle-Foot-Orthese (A-FO) dargestellt, mit einem Abstützbereich 10, einem Befestigungsmittel 20 und einem Auflagebereich 30, der mit dem Abstützbereich 10 über ein Verbindungselement 40 verbunden ist. Das Verbindungselement 40 ist im dargestellten Ausführungsbeispiel als formstabiles, im Wesentlichen starres Element ausgebildet, das nur eine geringfügige Verlagerung des Abstützbereiches 10 relativ zu dem Auflagebereich 30 zulässt. Alternativ dazu ist es überwiegend vorgesehen, dass der Auflagebereich 30 und der Abstützbereich 10 über eine gelenkige Verbindung miteinander gekoppelt sind, die beispielsweise als eine Feder ausgestaltet ist, bei der die überwiegende Verformung in der Sagittalebene bei einer Belastung in anterior-posterior-Richtung in einem Bereich um das natürliche Knöchelgelenk erfolgt. Der Auflagebereich 30 ist als flache Sohlenplatte ausgebildet, die im Wesentlichen der äußeren Kontur eines Fußes oder eines Schuhs folgt. Das dargestellte Ausführungsbeispiel ist aus einem faserverstärkten Kunststoff einstückig ausgebildet.

Der Abstützbereich 10 liegt im angelegten Zustand an dem Schienbein des Orthesennutzers an und umschließt das Schienbein medial und lateral zumindest teilweise. Die Kontur des Abstützbereiches 10 ist im Wesentlichen rinnenförmig oder schalenförmig und kann Ausnehmungen oder Durchbrechungen aufweisen, um eine verbesserte Belüftung des Schienbeinbereiches zu ermöglichen. Auf der Innenseite des Abstützbereiches 10, die im angelegten Zustand dem Orthesennutzer zugewandt ist, ist das Befestigungsmittel 20 angeordnet, das gleichzeitig mit einer Polsterung 25 versehen ist, so dass es nicht zu einer direkten Anlage der harten, schalenartigen Konstruktion des Abstützbereiches 10 an dem Unterschenkel kommt.

An dem Abstützbereich 10 sind sowohl medial als auch lateral Aufnahmebereiche 11, 12 in Gestalt von Vorsprüngen oder Laschen angeordnet, die in der Figur 1 nicht zu erkennen sind. Die Aufnahmebereiche 11, 12 oder Vorsprünge sind am proximalen Ende und distalen Ende des Abstützbereiches 10 ausgebildet und weisen im angelegten Zustand für den Orthesennutzer nach hinten. Nachfolgend wird von den Aufnahmebereichen 11, 12 als Vorsprünge gesprochen, worunter auch eine Ausbildung zu verstehen ist, bei der an den Abstützbereich 10 anschließende Ausformungen geeignet sind, in Taschen 21, 22 aufgenommen zu werden, um eine formschlüssige Festlegung der Polsterung zu bewirken. In der Figur 2 ist die Orthese 1 gemäß Figur 1 in einem geöffneten, nicht angelegten Zustand dargestellt, in dem auch die beiden als Vosprünge von einer vertikalen Linie des Abstützbereiches 10 ausgebildeten Aufnahmebereiche 12 auf der medialen Seite als Strichlinie gezeigt sind. Korrespondierende Aufnahmebereiche 11 und Taschen 21 sind auf der lateralen Seite des Abstützbereiches 10 vorgesehen.

An dem Befestigungsmittel 20 sind Taschen 21, 22 ausgebildet, in die die Vorsprünge 11, 12 eingeschoben sind. Durch das Einschieben der Vorsprünge 11, 12 in die Taschen 21, 22 wird das Befestigungsmittel 20 an dem Abstützbereich 10 festgelegt.

Dabei ist vorgesehen, dass die lateralen Taschen 21 tiefer als die medialen Taschen 22 sind, ebenso ist vorgesehen, dass die lateralen Vorsprünge 11 länger als die medialen Vorsprünge 12 sind, wobei die Tiefe der Taschen 21, 22 zu den Längen der Vorsprünge 11, 12 korrespondiert. Das Ausführungsbeispiels sieht medial und lateral jeweils zwei Taschen 21, 22 und zwei Vorsprünge 11, 12 vor, die in Längserstreckung versetzt zueinander angeordnet sind, so dass sich zwei proximale und zwei distale Paarungen von Taschen 21, 22 und Vorsprüngen 11, 12 ergeben. Alternativ kann auch nur jeweils eine Tasche medial und lateral vorgesehen und an einem korrespondierenden Aufnahmebereich festgelegt sein.

An dem Befestigungsmittel 20 sind sowohl am proximalen Rand als auch am distalen Rand des Abstützbereiches 10 Verbindungselemente 23, 24 vorgesehen, über die eine Festlegung der Orthese 1 an der Gliedmaße erfolgt. Die Verbindungselement 23, 24 sind auf der Medialseite an dem Befestigungsmittel 20 unter Ausbildung der medialen Taschen 22 befestigt, beispielsweise angeschweißt, angeklebt oder angenäht. Von der medialen Tasche 22 ist das als Gurt ausgeführte Verbindungselement 23, 24 außen auf der Außenseite des Abstützbereiches 10 entlang geführt und außen an der lateralen Tasche 21 festgelegt, beispielsweise durch einen Klettverschluss. Bei einer elastischen Ausgestaltung des gurtartigen Verbindungselementes 23, 24 werden die medialen und lateralen Taschen 21, 22 in Umfangsrichtung aufeinander zu mit einer Vorspannung beaufschlagt, so dass die nach vorne offenen Taschen 21, 22 nicht von den nach hinten weisenden Vorsprüngen 11, 12 heruntergleiten können. Zum weiteren Festlegen der Orthese 1 wird dann das jeweilige Verbindungselement 23, 24 um die nicht dargestellte Gliedmaße hinten herum von lateral nach medial geführt und dort auf einer Fixiereinrichtung 26, 27 festgelegt. Das Verbindungselement 23, 24 ist somit einmal um die Orthese und die Gliedmaße herumgeführt, wobei das freie Ende auf der Außenseite der medialen Tasche festgelegt wird. An dem freien Ende des Verbindungselementes 23, 24 sind beispielsweise Verriegelungselemente 28, 29 in Gestalt abnehmbarer Klettelemente befestigt, die auf der Außenseite des Verriegelungselementes 23, 24 bzw. auf Flauschbereichen der Fixiereinrichtungen 26, 27 außen auf den medialen Taschen 22 festgelegt werden können. Die Festlegung an den Verbindungselementen 23, 24 und auf den Fixiereinrichtungen 26, 27 erfolgt reversibel. Die Verbindungselemente 23, 24 können flexibel und unelastisch, flexibel und elastisch sowie nur bereichsweise elastisch sein, ebenfalls ist es möglich, dass das Verbindungselement 23, 24 reversibel an dem Befestigungsmittel 20 festgelegt ist, beispielsweise durch Klettverschlüsse.

Im angelegten Zustand, wie in der Figur 1 dargestellt, überbrückt das Verbindungselement 23, 24 den Freiraum innerhalb des schalenartigen Abstützbereiches 10 von lateral nach medial. Der Abstützbereich 10 kann eine Verformung der Vorsprünge 11, 12 in Richtung aufeinander zu zulassen, so dass eine Anpassung an die Abmessungen der Gliedmaße, an die die Orthese anzulegen ist, erfolgen kann. Die Einstellung kann dabei über die über die Verbindungselemente 23, 24 aufgebrachte Umfangskraft erfolgen.

Die dargestellte Ausführungsform ist besonders vorteilhaft für Patienten, die halbseitig gelähmt sind oder eine halbseitige Schwächung aufweisen. Wird das Verbindungselement 23 von medial nach lateral geführt, gedehnt und unter Spannung auf einer lateralen Tasche 21 festgelegt, kann über die nicht von der Schwächung betroffene Hand eine sichere Festlegung der Orthese dadurch erfolgen, dass das Verbindungselement 23, 24 anschließend hinter der Gliedmaße hindurchgeführt und eine Verriegelung auf der Medialseite erfolgt.

Das Befestigungsmittel 20 kann auch als einteiliges Element oder als ein mehrteiliges, dauerhaft miteinander verbundenes Element ausgebildet sein, das eine Polsterfunktion aufweist. Die medialen und lateralen Vorsprünge 11, 12 bilden autoadaptive Seitenflügel, die sich durch die Umfangskraft, die durch das Verbindungselement 23, 24 aufgebracht wird, selbstständig an die Form der Gliedmaße anpasst.

Durch Y-Klettbänder 28, 29 als Hakenbereiche ist es möglich, die als Gurte ausgeführten Verbindungselemente 23, 24 einfach zu kürzen, indem die Y-Klettbänder von den mit Flausch versehenden Verbindungselementen 23, 24 entfernt, die Länge auf das gewünschte Maß gekürzt und anschließend wieder aufgebracht werden.

Insbesondere bei Schlaganfallpatienten ist zu beachten, dass diese nur die nicht betroffene Körperseite zum Anziehen bzw. Anlegen der Orthese einsetzen können.

Durch die laterale Anordnung der Verbindungselemente 23, 24 und die Führung von lateral nach medial zum Befestigen kann der Orthesennutzer im Sitzen mit der nicht betroffenen Seite das Verbindungselement 23, 24 von lateral greifen und einfach nach medial gezogen und dort an den dort versehenen Haken- und Flauschbereichen 26, 27 festgelegt werden.

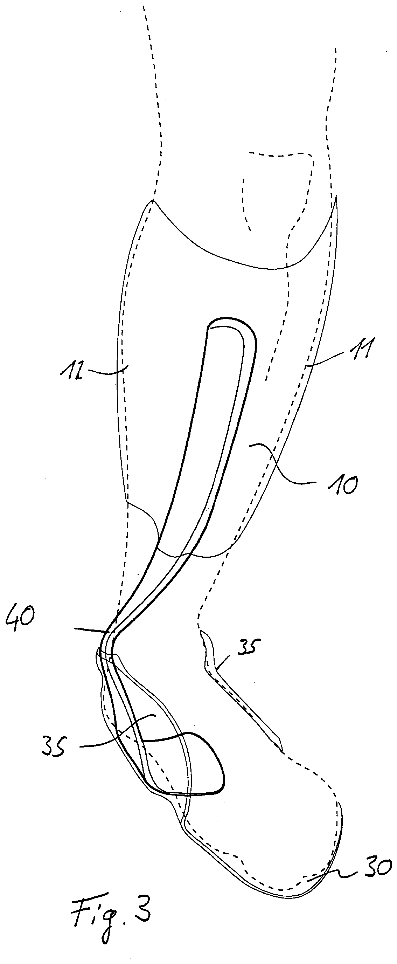

In der Figur 3 ist eine Variante der Erfindung dargestellt. Der Abstützbereich 10 und der Auflagebereich 30 sind durch ein separates Verbindungselement 40 miteinander verbunden. Der Auflagebereich 30 sieht neben einer flachen Sohle am Rand Erhöhungen 35 vor, die ein mediales und laterales Verrutschen des Fußes von dem Auflagebereich 30 verhindern. Im dargestellten Ausführungsbeispiel sind die Erhöhungen 35 im Bereich des Mittelfußes beginnend ausgebildet und erstrecken sich bis in den Fersenbereich, um gegebenenfalls ein Herausrutschen nach hinten zu vermeiden. Das Verbindungselement 40 ist als Feder ausgestaltet, um eine Verlagerung des Abstützbereiches 10 relativ zu dem Auflagebereich 30 zuzulassen, beispielsweise beim Gehen, wobei durch die Federwirkung des Verbindungselementes 40 der Fuß des Orthesennutzers nach Entlastung zurück in die Ausgangsposition bewegt wird. In dem Ausführungsbeispiel gemäß Figur 3 ist das Befestigungsmittel 20 gemäß der Figuren 1 und 2 nicht dargestellt. Im Gegensatz zu den medialen und lateralen Aufnahmebereichen 11, 12, die als Vorsprünge oder Laschen ausgebildet sind, ist im Ausführungsbeispiel gemäß Figur 3 der gesamte nach hinten weisende Rand des Abstützbereiches 10 als jeweiliger Aufnahmebereich 11, 12 ausgebildet. Die mediale und laterale Tasche 22, 21 des nicht dargestellten Befestigungsmittels 20 nimmt somit einen sich über die gesamte Höhe erstreckenden Rand des Abstützbereiches 10 auf, so dass insgesamt zwei Taschen 21, 22 auf der lateralen Seite des Befestigungsmittels 20 vorhanden sind, die die nach hinten zeigenden Enden des Abstützbereiches 10 aufnehmen. Die Verbindungselemente 23, 24 können an unterschiedlichen Höhen des Befestigungsmittels angeordnet sein, also ein proximales Verbindungsmittel 23 und ein distales Verbindungsmittel 24. Grundsätzlich ist es auch möglich, dass nur ein Verbindungsmittel vorgesehen ist, das von der medialen Seite frontal über den Abstützbereich und die laterale Seite des Abstützbereiches posterior um den Unterschenkel herum geführt wird, um dann wieder an der lateralen Seite des Befestigungsmittels 20 festgelegt zu werden.

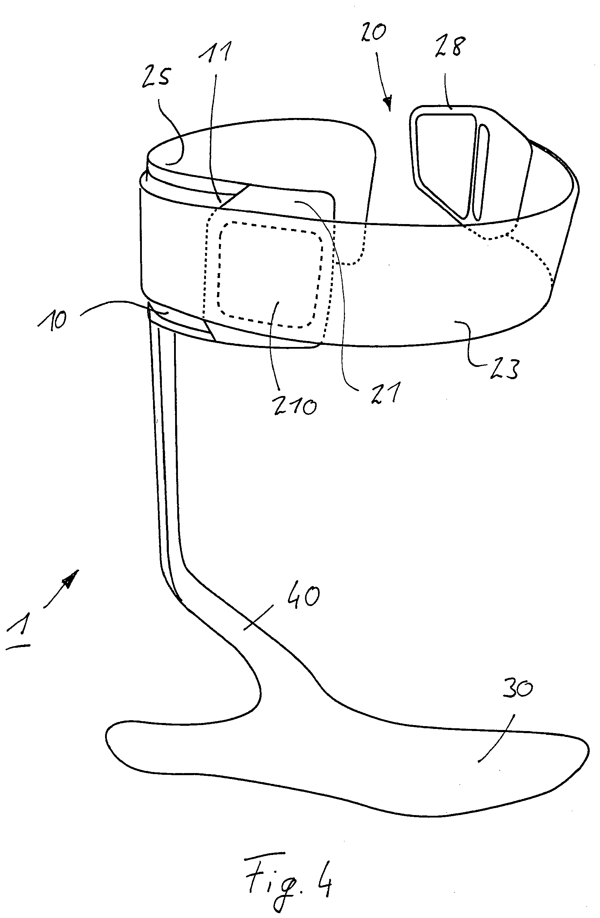

In der Figur 4 ist eine weitere Variante der Erfindung dargestellt, bei der die Orthese 1 einen Auflagebereich 30 in Gestalt einer flachen Sohle für einen Fuß oder Schuh, ein Federelement als Verbindungselement 40 und einen Abstützbereich 10 aufweist, der dorsal ausgeführt ist, so dass eine Anlage des Abstützbereiches 10 an der Wade und nicht an dem Schienbein des Orthesennutzers erfolgt. Die Orthese 1 ist im dargestellten Ausführungsbeispiel einteilig ausgebildet, grundsätzlich besteht die Möglichkeit, dass das Verbindungselement 40, der Auflagebereich 30 und der Abstützbereich 10 separat ausgeführt und zur Montage der Orthese 1 miteinander verbunden sind.

Auf der dem Nutzer zugewandten Innenseite des Abstützbereiches 10 ist die Polsterung 25 angeordnet, die sich vor den U-förmig gebildeten, nach vorne offenen Abstützbereich 10 legt und die gegebenenfalls scharfen Kanten des formstabilen Materials abpolstert. Die Polsterung 25 kann an den Rändern des Abstützbereiches 10 überstehen.

Das Verbindungselement 40 ist medial an dem Auflagebereich angeordnet und verläuft vom Fußgewölbe schräg nach dorsal und proximal, also schräg nach hinten und oben und wird dorsal an dem Bein nach oben geführt, wobei der Knöchelbereich vorteilhafterweise freigelassen wird.

Das Befestigungsmittel 20 ist wie in den oben beschriebenen Ausführungsformen über Taschen 21, 22 an Aufnahmebereichen 11, 12 des Abstützbereiches 10 festgelegt. In der Figur 4 sind nur der laterale Aufnahmebereich 11 und die laterale Tasche 21 gezeigt. Auf der medialen Seite ist das vordere Ende des Abstützbereiches 10 in einer Tasche des Befestigungsmittels 20 eingeführt. Die mediale Tasche ist aus der Polsterung 25 und dem darauf aufgenähten, aufgeschweißten oder aufgeklebten Verbindungselement 23 gebildet. Das Verbindungselement 23 ist dann dorsal auf der Außenseite des Abstützbereiches 10 herumgeführt und an einem Klettverschluss 210 auf der Außenseite der lateralen Tasche 21 fixiert. Diese Fixierung kann unter einer Vorspannung des elastisch ausgebildeten Verbindungselementes 23 erfolgen, so dass das Befestigungsmittel 20 über die Taschen 21 an den Abstützbereichen 11 sicher und dauerhaft festgelegt ist. Der dann noch offene Einstieg für den Unterschenkel wird dann durch das Verbindungselement 23 geschlossen, der über das Schienbein des Orthesennutzers hinweg geführt und mit dem Verriegelungselement 28 auf der medialen Außenseite des Befestigungsmittels 20, beispielsweise einem Flauschbereich auf der Außenseite der medialen Tasche, festgelegt wird.

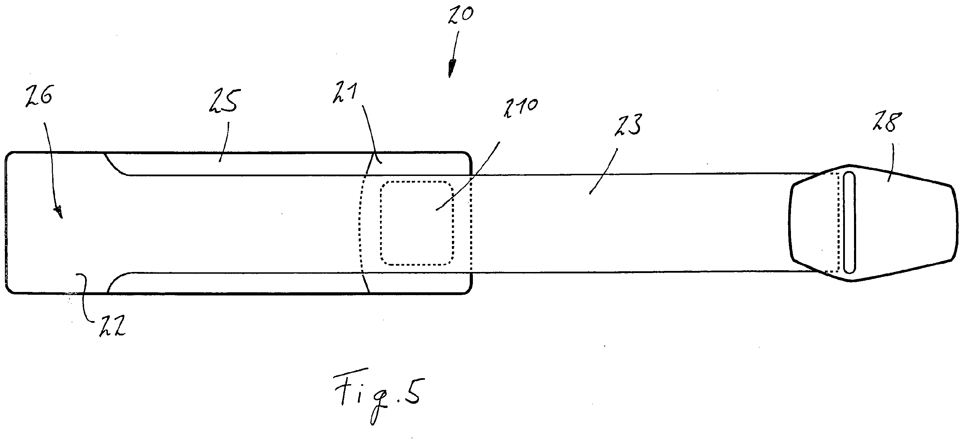

Figur 5 zeigt das Befestigungsmittel 20 in Einzeldarstellung flach ausgebreitet. Die Polsterung 25 als Basis ist mit dem Verbindungselement 23 auf der Außenseite versehen. Das Verbindungselement 23 weist an seinem in der Figur 5 linken Ende eine Verbreitung auf, die der Breite der Polsterung 25 entspricht. Im Bereich dieser Verbreiterung ist das Verbindungselement 23 an der Polsterung 25 an den Rändern befestigt, so dass eine Tasche 22 zur Aufnahme des nicht dargestellten Aufnahmebereiches ausgebildet wird. Auf der Außenseite der Tasche 22 kann eine Fixiereinrichtung 26 in Gestalt eines Klettverschlusses oder eines Flauschbereiches angeordnet und ausgebildet sein, um das an dem gegenüberliegenden Ende befestigte Verriegelungselement 28 außenseitig darauf festzulegen.

Von der Tasche 22 erstreckt sich das Verbindungselement 23 in Richtung auf das andere Ende der Polsterung 25, an der eine separate Tasche 21 durch Aufnähen, Aufschweißen oder Kleben eines Textils oder dergleichen ausgebildet ist. Auf der Außenseite der Tasche 21, die im angelegten Zustand lateral angeordnet ist, ist ein Verschlusselement 210 in Gestalt eines Klettverschlusses aufgebracht, befestigt oder ausgebildet, um das beispielsweise mit einer Flauschschicht versehene Verbindungselement 23 daran festzulegen. An dem Ende des Verbindungselementes 23 ist über einen weiteren Klettverschluss das Verriegelungselement 23 festgelegt, das als Y-Element ausgebildet ist, so dass das beidseitig mit einer Flauschschicht versehene Verbindungselement 23 von den als Hakenelemente ausgebildeten Verschlusselementen an dem Verriegelungselement 28 aufgenommen und festgelegt werden kann.

## Patentansprüche

1. Orthese mit einem Abstützbereich (10), der Befestigungsmittel (20) aufweist, um die Orthese (1) an einer Gliedmaße anzulegen, wobei der Abstützbereich einander entgegengesetzt orientierte Aufnahmebereiche (11, 12) aufweist, wobei die Aufnahmebereiche (11, 12) medial und lateral vorgesehen sind, **dadurch gekennzeichnet, dass** an dem Befestigungsmittel (20) Taschen (21, 22) zur Aufnahme der Aufnahmebereiche (11, 12) angeordnet sind, die Taschen (21, 22) über ein reversibel festlegbares Verbindungselement (23, 24) miteinander verbunden sind und das Verbindungselement (23, 24) als ein den Abstützbereich (10) und die Gliedmaße umschlingender Gurt ausgebildet ist.

2. Orthese nach Anspruch 1, **dadurch gekennzeichnet, dass** das Befestigungsmittel (20) Teil einer Polsterung (25) ist, die auf der dem Nutzer zugewandten Seite der Orthese (1) angeordnet ist.

3. Orthese nach Anspruch 1 oder 2, **dadurch gekennzeichnet, dass** die Taschen (21, 22) medial und lateral angeordnet sind.

4. Orthese nach Anspruch 3, **dadurch gekennzeichnet, dass** die lateral angeordneten Taschen (21) und/oder Aufnahmebereiche (11) länger als die medial angeordneten Taschen (22) und/oder Aufnahmebereiche (12) sind.

5. Orthese nach einem der voranstehenden Ansprüche, **dadurch gekennzeichnet, dass** der Abstützbereich (10) schalenartig oder rinnenartig ausgebildet ist.

6. Orthese nach Anspruch 1, **dadurch gekennzeichnet, dass** das Verbindungselement (23, 24) elastisch ausgebildet ist und eine Vorspannung der Taschen (21, 22) in Richtung aufeinander zu ermöglicht.

7. Orthese nach einem der voranstehenden Ansprüche, **dadurch gekennzeichnet, dass** das Verbindungselement (23, 24) Teil des Befestigungsmittels (20) ist.

8. Orthese nach einem der voranstehenden Ansprüche, **dadurch gekennzeichnet, dass** auf der Außenseite zumindest einer Tasche (21, 22) eine Fixiereinrichtung (26, 27) für das Verbindungselement (23, 24) angeordnet ist.

9. Orthese nach einem der voranstehenden Ansprüche, **dadurch gekennzeichnet, dass** an dem Befestigungsmittel (10) Flausch- und Hakenbereiche (26, 27, 28, 29) vorhanden sind.

10. Orthese nach einem der voranstehenden Ansprüche, **dadurch gekennzeichnet, dass** das Verbindungselement (23, 24) einen Freiraum (15) zwischen den Aufnahmebereichen (11, 12) von lateral nach medial überbrückt.

11. Orthese nach einem der voranstehenden Ansprüche, **dadurch gekennzeichnet, dass** der Abstützbereich (10) eine Längserstreckung aufweist und elastisch um eine Achse parallel zu der Längserstreckung ausgebildet ist.

12. Orthese nach einem der voranstehenden Ansprüche, **dadurch gekennzeichnet, dass** ein Auflagebereich (30) der Orthese (1) mit dem Abstützbereich (10) verbunden ist und sich an der Gliedmaße abstützt.

13. Orthese nach Anspruch 12, **dadurch gekennzeichnet, dass** der Auflagebereich (30) und der Abstützbereich (10) gelenkig miteinander verbunden sind.

14. Orthese nach einem der voranstehenden Ansprüche, **dadurch gekennzeichnet, dass** die Aufnahmebereiche (11, 12) als Vorsprünge ausgebildet sind.

## Claims

1. An orthosis with a supporting region (10) that has fastening means (20) for fitting the orthosis (1) on a limb, wherein the supporting region (10) has receiving regions (11, 12) oriented opposite to each other, wherein the receiving regions (11, 12) are arranged medially and laterally, **characterized in that** pockets (21, 22) for receiving the receiving regions (11, 12) are arranged on the fastening means (20), that the pockets (21, 22) are connected to each other via the reversibly attachable connection element (23, 24), and that the connection element (23, 24) is designed as a strap looping around the supporting region (10) and the limb.

2. The orthosis as claimed in claim 1, **characterized in that** the fastening means (20) is part of a padding (25) which is arranged on the side of the orthosis (1) facing toward the user.

3. The orthosis as claimed in claim 1 or 2, **characterized in that** the pockets (21, 22) are arranged medially and laterally.

4. The orthosis as claimed in claim 3, **characterized in that** the laterally arranged pockets (21) and/or receiving regions (11) are longer than the medially arranged pockets (22) and/or receiving regions (12).

5. The orthosis as claimed in one of the preceding claims, **characterized in that** the supporting region (10) has a cup-like or gutter-like shape.

6. The orthosis as claimed in claim 1, **characterized in that** the connection element (23, 24) is elastic and allows the pockets (21, 22) to be pretensioned toward each other.

7. The orthosis as claimed in one of the preceding claims, **characterized in that** the connection element (23, 24) is part of the fastening means (20).

8. The orthosis as claimed in one of the preceding claims, **characterized in that** a fixing means (26, 27) for the connection element (23, 24) is arranged on the outer side of at least one pocket (21, 22).

9. The orthosis as claimed in one of the preceding claims, **characterized in that** hook and loop regions (26, 27, 28, 29) are present on the fastening means (10).

10. The orthosis as claimed in one of the preceding claims, **characterized in that** the connection element (23, 24) forms a lateral to medial bridge across a gap (15) between the receiving regions (11, 12).

11. The orthosis as claimed in one of the preceding claims, **characterized in that** the supporting region (10) has a longitudinal extent and is designed elastically about an axis parallel to the longitudinal extent.

12. The orthosis as claimed in one of the preceding claims, **characterized in that** a resting region (30) of the orthosis (1) is connected to the supporting region (10) and bears on the limb.

13. The orthosis as claimed in claim 12, **characterized in that** the resting region (30) and the supporting region (10) are connected to each other in an articulated manner.

14. The orthosis as claimed in one of the preceding claims, **characterized in that** the receiving regions (11, 12) are designed as projections.

## Revendications

1. Orthèse comprenant une zone d'appui (10) munie de moyens de fixation (20) pour appliquer l'orthèse (1) contre un membre, la zone d'appui (10) comprenant des zones de réception (11, 12) orientées de manière opposée l'une à l'autre, les zones de réception (11, 12) étant prévues du côté médial et du côté latéral,
**caractérisée en ce que** des poches (21, 22) destinées à recevoir les zones de réception (11, 12) sont disposées sur le moyen de fixation (20), **en ce que** les poches (21, 22) sont reliées entre elles par un élément de liaison (23, 24) fixable de manière réversible, et **en ce que** l'élément de liaison (23, 24) est réalisée sous la forme d'une sangle s'enroulant autour de la zone d'appui (10) et du membre.

2. Orthèse selon la revendication 1,
**caractérisée en ce que** le moyen de fixation (20) fait partie d'un rembourrage (25) disposé sur le côté de l'orthèse (1) tourné vers l'utilisateur.

3. Orthèse selon la revendication 1 ou 2,
**caractérisée en ce que** les poches (21, 22) sont disposées du côté médial et du côté latéral.

4. Orthèse selon la revendication 3,
**caractérisée en ce que** les poches (21) et/ou les zones de réception (11) disposées du côté latéral sont plus longues que les poches (22) et/ou les zones de réception (12) disposées du côté médial.

5. Orthèse selon l'une des revendications précédentes,
**caractérisée en ce que** la zone d'appui (10) est réalisée en forme de coque ou de rainure.

6. Orthèse selon la revendication 1,
**caractérisée en ce que** l'élément de liaison (23, 24) est réalisé élastique et permet une précontrainte des poches (21, 22) l'une vers l'autre.

7. Orthèse selon l'une des revendications précédentes,
**caractérisée en ce que** l'élément de liaison (23, 24) fait partie du moyen de fixation (20).

8. Orthèse selon l'une des revendications précédentes,
**caractérisée en ce qu'**un organe de fixation (26, 27) pour l'élément de liaison (23, 24) est disposé sur le côté extérieur d'au moins une poche (21, 22).

9. Orthèse selon l'une des revendications précédentes,
**caractérisée en ce que** des zones à bouclettes et à crochets (26, 27, 28, 29) sont prévues sur le moyen de fixation (10).

10. Orthèse selon l'une des revendications précédentes,
**caractérisée en ce que** l'élément de liaison (23, 24) ponte un espace libre (15) entre les zones de réception (11, 12) du côté latéral vers le côté médial.

11. Orthèse selon l'une des revendications précédentes,
**caractérisée en ce que** la zone d'appui (10) présente une extension longitudinale et est formée élastiquement autour d'un axe parallèle à l'extension longitudinale.

12. Orthèse selon l'une des revendications précédentes,
**caractérisée en ce qu'**une zone de support (30) de l'orthèse (1) est reliée à la zone d'appui (10) et est en appui contre le membre.

13. Orthèse selon la revendication 12,
**caractérisée en ce que** la zone de support (30) et la zone d'appui (10) sont reliées l'une à l'autre de manière articulée.

14. Orthèse selon l'une des revendications précédentes,
**caractérisée en ce que** les zones de réception (11, 12) sont réalisées sous forme de saillies.
